(19) Europäisches Patentamt
European Patent Office
Office européen des brevets

(11) Publication number: 0 308 235 B1

(12) EUROPEAN PATENT SPECIFICATION

(45) Date of publication of patent specification: 22.07.92

(51) Int. Cl.[5]: G01N 33/547, G01N 33/545, G01N 33/546, C12N 11/08

(21) Application number: 88308566.4

(22) Date of filing: 16.09.88

# (54) Attachment of compounds to polymeric particles using carbamoylonium compounds.

(30) Priority: 18.09.87 US 98429

(43) Date of publication of application:
22.03.89 Bulletin 89/12

(45) Publication of the grant of the patent:
22.07.92 Bulletin 92/30

(84) Designated Contracting States:
CH DE FR GB LI

(56) References cited:
WO-A-83/00345
US-A- 4 181 636
US-A- 4 415 700
US-A- 4 434 228
CH-A- 650 592
US-A- 4 401 765
US-A- 4 421 847

(73) Proprietor: EASTMAN KODAK COMPANY (a New Jersey corporation)
343 State Street
Rochester New York 14650(US)

(72) Inventor: Sutton, Richard Calvin, c/o Eastman Kodak Co.,
Patent Dept., 343 State Street
Rochester, NY 14650(US)
Inventor: Danielson, Susan Jean, c/o Eastman Kodak Co.,
Patent Dept., 343 State Street
Rochester, NY 14650(US)
Inventor: Bagchi, Pranab, c/o Eastman Kodak Co.,
Patent Dept., 343 State Street
Rochester, NY 14650(US)

(74) Representative: Nunney, Ronald Frederick Adolphe et al
Kodak Limited Patent Department Headstone Drive
Harrow Middlesex HA1 4TY(GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 308 235 B1

## Description

The present invention relates to a method for the preparation of polymeric particles having compounds attached thereto. In particular, it relates to the preparation of such materials by attachment of reactive amine- or sulfhydryl-containing compounds to polymeric particles using carbamoylonium compounds.

Biologically active polypeptides or proteins which are attached to insoluble carrier materials, such as polymeric particles, have been used in a variety of ways. For example, the diagnosis of pathological or other conditions in human beings and animals is often carried out using immunological principles for the detection of an immunologically reactive species, for example antibodies or an antigen, in the body fluids of the person or animal. An antigen is generally known as a foreign substance, such as a drug, hapten, toxin, lectin, polypeptide or protein which, when introduced into the body, causes the production of certain soluble proteins known as antibodies.

Other proteins and amine-containing compounds, such as enzymes, avidin, biotin or polysaccarides, have been covalently linked to various carrier materials for use in affinity chromatography, enzymatic reactions, specific binding reactions and immunoassays. Among useful carrier materials are sheep and human erythrocytes, bacterial cells, latex particles, resinous particles and finely divided diazotized amino cellulose. For example, carrier particles prepared from sparingly water-soluble monomers (such as epoxy group-containing monomers) in the absence of emulsifiers are known in the art. Other compounds, such as diamines, dihydrazides, mercaptoalkylamines and dimercaptans have been attached to carrier materials as linking moieties for later attachment of drugs, enzymes or other reactive species.

Carboxylated latex particles have also been used to prepare diagnostic reagents as described, for example, in U.S. Patent 4,181,636. As described therein, the procedure for covalently attaching an immunologically reactive species to the particles having surface carboxyl groups involves the use of a water-soluble carbodiimide. While producing useful reagents, this procedure tends to activate the exposed reactive groups of the reactive species as well as the carboxyl groups. The result is intramolecular and intermolecular crosslinking or polymerization of the immunologically reactive species, and a significant portion of the species is thus impaired from complexation with a receptor molecule. Because the reactive species, for example an antibody, is usually very costly, this problem represents a serious economic loss. It has also been evident that the use of carbodiimides to attach proteins to carrier particles is not as efficient as desired at certain protein levels.

It would be desirable to have a rapid method for attaching a reactive amine-containing compound to carboxylated polymeric particles in an efficient manner and without adversely affecting the attached compound.

The problems noted above are overcome with a method for attaching a reactive amine- or sulfhydryl-containing compound to polymeric particles having pendant carboxyl groups,

the method characterized wherein it comprises

A. contacting (1) an aqueous suspension of polymeric particles having carboxyl groups on the surface thereof, the particles prepared from one or more ethylenically unsaturated polymerizable monomers, at least one of which contains pendant carboxyl groups with (2) a carbamoylonium compound to produce reactive intermediate polymer particles having pendant intermediate reactive groups, and

B. contacting the reactive intermediate polymer particles produced in step A with a reactive amine- or sulfhydryl-containing compound having a reactive amino or sulfhydryl group, respectively, which reacts with the intermediate reactive groups to form a covalent linkage between the particles and the reactive compound.

The present invention provides a means for rapidly attaching a reactive amine- or sulfhydryl-containing compound, such as a biologically active polypeptide or protein to insoluble polymeric particles, thereby forming useful materials for immunoassays, diagnostic tests, affinity chromatography, enzymatic reactions and other biological or chemical procedures. The attachment is achieved without adversely affecting the reactive compound which is attached. That is, there is minimal crosslinking or deactivation of the reactive amino or sulfhydryl groups in the reactive compound which participate in the formation of a covalent linkage with pendant carboxyl groups of the particles.

These advantages are achieved by using a particular class of attachment agents. These agents are carbamoylonium compounds which have not been heretofore used for this purpose. It was initially expected that such compounds, like the conventional carbodiimides, would indiscriminately deactivate the reactive amine or sulfhydryl groups of the reactive compounds. Unexpectedly, we found this not to be the case, and thereby discovered the very useful and efficient attachment method described and claimed herein.

The materials prepared according to the method of the present invention can be used in many different chemical and biological procedures. For example, they can be used in affinity chromatography, reactions

catalyzed by enzymes, water purification, immunoassays wherein the analyte is an immunologically reactive species which has specific binding affinity for an attached polypeptide or protein, and other processes known to one of ordinary skill in the art. In some instances, the present invention can be used to attach intermediate linking moieties which can be further reacted with compounds of biological interest, such as drugs, hormones, enzymes, antibodies or other proteins or polysaccharides.

One use of the materials prepared by the present invention is as an agglutination immunochemical reagent in an agglutination assay wherein the analyte or material to be detected is an immunologically reactive species found in physiological fluids, cells or tissue extracts of humans or animals, for which an immunological counterpart (or receptor) is available or can be produced. Representative immunologically reactive species for which the reagent can be used to detect include, but are not limited to, microorganisms (bacteria, protozoa, fungi, viruses and rickettsia), tissue antigens including organ specific antigens, hormones, enzymes, blood cell antigens or other substances found in the blood, plasma proteins, milk proteins, saliva proteins, urine proteins, pathologic proteins, antibodies including autoantibodies and drugs. In such instances, the reactive amine- or sulfhydryl-containing compound used in the method of this invention is an immunological compound which is a receptor for the analyte of interest, for example an antigen or antibody.

In other embodiments, the material described herein can have an enzyme attached to the particles. Enzymes which can be attached in this manner include those which have reactive amine or sulfhydryl groups which can be reacted according to the present invention with the active pendant groups on the particles without losing enzymatic activity.

In still other embodiments, the material prepared by this invention can be used in competitive binding assays in either a solution or dry format (that is, a dry analytical element), or in what are known in the art as immunometric assays, for example "sandwich" assays.

The method of this invention is a two-step process involving attaching a reactive amine- or sulfhydryl-containing compound which has a reactive amine or sulfhydryl group, respectively, to polymeric particles having reactive carboxyl groups using a carbamoylonium compound.

The polymeric particles useful in the method of this invention are generally water-insoluble latex particles having a particle size in the range of from 0.01 to 5 micrometers, and preferably from 0.1 to 3 micrometers. They can be homogeneous polymeric particles meaning that they are composed of the same polymer throughout, or they can be particles composed of more than one polymer such as graft copolymers as described, for example, in U.S. Patent 3,700,609 and core-shell polymers described for example in U.S. Patent 4,401,765. It is critical that the polymeric particles have surface carboxyl groups available for attachment of the reactive amine- or sulfhydryl-containing compound. Such groups can be added to the particles by incorporating monomers containing such groups into the polymers (for example, acrylic acid, methacrylic acid, itaconic acid), or by further chemical reaction of a polymer having other reactive groups which can be converted to carboxyl groups (for example, by hydrolysis of anhydrides, such as maleic anhydride, or by oxidation of surface methylol or aldehyde end groups).

The polymeric particles can be prepared using any suitable polymerization technique, including emulsion (including batch, semi-continuous and continuous) and suspension polymerization techniques, graft copolymerization, and others known to one skilled in the polymer chemistry art. Emulsion polymerization is preferred as it can be used to provide generally smaller particles without the use of surfactants or emulsifiers as described for example in U.S. Patent 4,415,700 (noted above) and Research Disclosure publication 15963 (July, 1977). Continuous emulsion polymerization is the most preferred technique, as described in the noted Research Disclosure publication.

Useful carboxylated particles are prepared from carboxylated styrene and its derivatives, carboxylated styrene-butadiene copolymers, acrylic and methacrylic acid polymers and other materials, many of which are commercially available.

Preferably, the polymeric particles are composed of a polymer represented by the structure:

$$-(-A-)_x-(-B-)_{100-x}$$

wherein A represents recurring units derived from one or more ethylenically unsaturated polymerizable monomers containing carboxylic acid groups or salts or precursors of said groups, and B represents recurring units derived from one or more ethylenically unsaturated polymerizable monomers.

Monomers from which A can be derived include, but are not limited to, acrylic and methacrylic acids, itaconic acid, aconitic acid, fumaric acid, maleic acid, β-carboxyethyl acrylate, β-carboxyethyl methacrylate, m&p-carboxymethylstyrene, methacrylamidohexanoic acid and N-(2-carboxy-1,1,-dimethylethyl)acrylamide

or a salt or anhydride precursor thereof. Acrylic and methacylic acids, itaconic acid, aconitic acid, fumaric acid, maleic acid, β-carboxyethyl acrylate, β-carboxyethyl methacrylate or a salt or anhydride precursor thereof are preferred in the practice of this invention. Monomers from which B can be derived include, but are not limited to, styrene and styrene derivatives (for example vinyltoluene, 4-t-butylstyrene, divinylbenzene and 2-chloromethylstyrene), acrylic and methacrylic acid esters (for example, methyl acrylate, ethyl methacrylate, n-butyl acrylate, 2-ethylhexyl methacrylate, methyl methacrylate, 2-hydroxyethyl methacrylate, methacrylamide, ethylene dimethacrylate and 2-hydroxyethyl acrylate), sodium 2-acrylamido-2-methylpropansulfonate, sodium 3-acryloyloxypropanesulfonate, p-styrenesulfonate, or acrylonitrile. Preferably, B is derived from styrene or a styrene derivative, or an acrylic or methacrylic acid ester.

For both the A and B monomers, it is important that the specific monomers used and their proportions be chosen so as to render the particles water-insoluble.

In the structure identified above, x is from 0.1 to 70, and preferably from 1 to 20, mole percent.

Representative polymers of which the polymeric particles are composed include poly(styrene-co-vinylbenzyl chloride-co-acrylic acid) (85:10:5 molar ratio), poly(styrene-co-acrylic acid) (99:1 molar ratio), poly(styrene-co-methacrylic acid) (90:10 molar ratio), poly(styrene-co-acrylic acid-co-m&p-divinylbenzene) (89:10:1 molar ratio), poly(styrene-co-2-carboxyethyl acrylate) (90:10 molar ratio) and poly(methyl methacrylate-co-acrylic acid) (70:30 molar ratio).

In one embodiment, the particles are core-shell particles wherein the core is composed of a first polymer, and the shell is composed of a second polymer. The second polymer must have reactive carboxyl groups or groups which can be converted to carboxyl groups prior to attachment of the polypeptide or protein. A representative example of core-shell polymeric particles is provided in Example 2 below.

Carbamoylonium salts are used for covalent attachment of the reactive amine- or sulfhydryl-containing compound to the polymeric particles in the practice of this invention. These salts are described in some detail in U.S. Patent 4,421,847, and are generally represented by the structure:

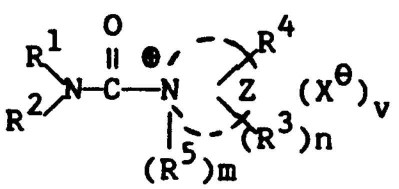

(I)

In structure (I), Z represents the atoms necessary to complete a substituted or unsubstituted 5- or 6-membered heterocyclic aromatic ring including heterocyclic rings having a fused carbocyclic ring (for example a pyridinium, imidazolium, thiazolium, isoxazolium or quinolinium ring). Preferably, Z represents the atoms necessary to complete a substituted 6-membered heterocyclic aromatic ring.

Further, m and n are independently 0 or 1,

$R^1$ and $R^2$ are, independently of each other, substituted or unsubstituted alkyl or substituted or unsubstituted aryl. Preferred alkyl groups have from 1 to 6 carbon atoms, for example, methyl, ethyl and isopropyl. Examples of substituted alkyl groups are chloromethyl and substituted or unsubstituted aralkyl groups (preferably having from 7 to 12 carbon atoms, for example, benzyl or phenethyl). Preferred aryl groups have from 6 to 10 carbon atoms, for example, phenyl and naphthyl. Examples of substituted aryl groups are p-methylphenyl and m-chlorophenyl.

Alternatively, $R^1$ and $R^2$ together represent the atoms necessary to complete a piperidine, piperazine or morpholine ring, which ring can be substituted for example with one or more alkyl groups each having 1 to 3 carbon atoms or by a halogen atom.

$R^3$ is a hydrogen atom, a substituted or unsubstituted alkyl as defined above for $R^1$, or the group

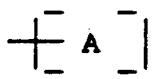

wherein A represents the polymerized vinyl backbone of a homo- or copolymer formed from one or more ethylenically unsaturated polymerizable compounds such that the molecular weight of the homo- or copolymer is greater than 1000. Useful ethylenically unsaturated polymerizable compounds are known to one of ordinary skill in the polymer chemistry art. The polymer [A] can comprise additional moieties derived from the compounds represented by structure (I).

$R^4$ is a hydrogen atom, a substituted or unsubstituted alkyl (as defined above for $R^1$), or when Z

represents the atoms necessary to complete a pyridinium ring and n is 0, $R^4$ is selected from the following groups:

(a) $-NR^6-CO-R^7$ wherein $R^6$ is hydrogen or substituted or unsubstituted alkyl (preferably of 1 to 4 carbon atoms, for example methyl ethyl, n-butyl, chloromethyl), $R^7$ is hydrogen, substituted or unsubstituted alkyl (as defined above for $R^6$) or $=NR^8R^9$ wherein $R^8$ and $R^9$ are independently of each other hydrogen or substituted or unsubstituted alkyl (as defined above for $R^6$),

(b) $-(CH_2)_q-NR^{10}R^{11}$ wherein $R^{10}$ is $-CO-R^{12}$, $R^{11}$ is hydrogen or substituted or unsubstituted alkyl (as defined above for $R^6$), $R^{12}$ is hydrogen, substituted or unsubstituted alkyl (as defined above for $R^6$) or $-NR^{13}R^{14}$ wherein $R^{13}$ is substituted or unsubstituted alkyl (as defined above for $R^6$) or substituted or unsubstituted aryl (as defined above for $R^1$), $R^{14}$ is hydrogen, substituted or unsubstituted alkyl (as defined above for $R^6$) or substituted or unsubstituted aryl (as defined for $R^1$), and q is 1 to 3,

(c) $-(CH_2)_r-CONR^{15}R^{16}$ wherein $R^{15}$ is hydrogen, substituted or unsubstituted alkyl (as defined above for $R^6$) or substituted or unsubstituted aryl (as defined above for $R^1$), $R^{16}$ is hydrogen or substituted or unsubstituted alkyl (as defined above for $R^6$), or $R^{15}$ and $R^{16}$ together represent the atoms necessary to complete a 5- or 6-membered aliphatic ring, and r is 0 to 3,

(d) 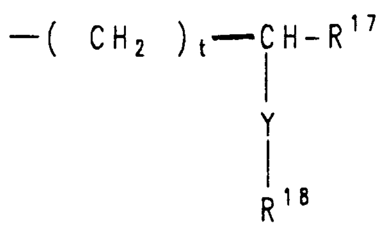

wherein $R^{17}$ is hydrogen, substituted or unsubstituted alkyl (as defined above for $R^6$), Y is oxy or $-NR^{19}-$, $R^{18}$ is hydrogen, substituted or unsubstituted alkyl (as defined above for $R^6$), $-CO-R^{20}$ or $-CO-NHR^{21}$ wherein $R^{19}$, $R^{20}$ and $R^{21}$ are independently of each other hydrogen or substituted or unsubstituted alkyl (as defined above for $R^6$), and t is 2 or 3, and

(e) $-R^{21}X'^{\ominus}$ wherein $R^{21}$ is substituted or unsubstituted alkylene of from 1 to 6 carbon atoms (for example, methylene, trimethylene or isopropylene), and $X'^{\ominus}$ is a covalently bonded anionic group such as sulfonate or carboxylate so as to form an inner salt group with the pyridinium nucleus.

$R^5$ is substituted or unsubstituted alkyl (as defined above for $R^6$), or substituted or unsubstituted aryl (as defined above for $R^1$) , provided that m is 0 when the nitrogen atom to which $R^5$ is bound is attached to the remainder of the ring through a double bond.

$X^{\ominus}$ is an anion, such as a halide, tetrafluoroborate, nitrate, sulfate, p-toluenesulfonate, perchlorate, methosulfate or hydroxide, and v is 0 or 1, provided that it is 0 only when $R^4$ is $-R^{21}X'^{\ominus}$.

Preferably, the carbamoylonium compound used in the practice of this invention is represented by the structure above wherein $R^1$ and $R^2$ together represent the atoms necessary to complete a morpholine ring, Z represents the atoms necessary to complete a pyridinium ring, $R^4$ is $-R^{21}X'^{\ominus}$ (such as $-CH_2CH_2SO_3^-$ ), and m, n and v are each 0.

Representative carbamoylonium compounds include 1-(4-morpholinocarbonyl)-4-(2-sulfoethyl)pyridinium hydroxide, inner salt, and 1-(4-morpholinocarbonyl)pyridinium chloride.

Most preferred is 1-(4-morpholinocarbonyl)-4-(2-sulfoethyl)pyridinium hydroxide, inner salt.

Any reactive amine- or sulfhydryl-containing compound can be attached to polymeric particles according to the present invention as long as that compound contains a reactive amine or sulfhydryl group, respectively, which will react with the intermediate formed by the reaction of the carbamoylonium compound with carboxyl groups on the particles. Such compounds include, but are not limited to, monoamines, monohydrazides, diamines, dihydrazides, enzymes, biotin or derivatives thereof, avidin or derivatives thereof, amino acids, peptides, polypeptides, proteins, polysaccharides, and others which would be apparent to one skilled in the art. In certain embodiments, the reactive amine- or sulfhydryl-containing compound is a polypeptide or protein which is biologically active. The term "biologically active" refers to its capacity for interaction with another component which may be found in physiological fluids. Such an interaction can be catalytic activity in the case where the material is an enzyme. In addition, the interaction can be a complexation which occurs between materials which have affinity for one another, such as avidin with biotin or antibodies with antigens. In other embodiments, the reactive amine- or sulfhydryl-containing compound is a diamine, polysaccharide, amino acid, peptide or protein which can be a linking moiety for attaching a second compound to the particle. Such second compounds include, but are not limited to,

enzymes, antibodies, antigens, drugs, biotin or derivatives thereof and others readily apparent to one skilled in the art.

Preferably, the reactive amine- or sulfhydryl-containing compound is an immunologically reactive species, including but not limited to the biological and chemical compounds listed above. More preferably, it is an antibody, such as an antibody directed against a drug, hormone, Streptococcus A antigen, a chlamydial antigen, a gonococcal antigen, human chorionic gonadotropin, human luteinizing hormone or a herpes virus. Alternatively, the immunologically reactive species can be an antigen, such as an antigen of HTLV-I or HIV-I.

In certain embodiments, the materials prepared by the method of this invention can have a tracer associated therewith. A tracer is a detectable species which enables one to detect the reagent. Useful tracers include radioisotopes, colorimetric or fluorometric compounds, enzymes, chemiluminescent compounds, phosphorescent compounds and others known to one skilled in the art. The tracer can be associated with the reagent in any suitable manner. For example, the tracer can be associated (for example, covalently or ionically attached) with the biologically active polypeptide or protein. Alternatively and preferably, the tracer is associated with the polymeric particles, for example attached (covalently or absorbed) to their outer surface or internally distributed in part or all of the volume, or both.

In one embodiment, the tracer is distributed in the polymer particles, either throughout or on its surfaces, by means of colored moieties or color coupler moieties. Colored moieties are attached to the backbone of the polymers of which the particles are composed. Color coupler moieties are capable of being oxidatively coupled with a color developing compound to provide a dye. These moieties are also attached to the polymer backbone, and are provided by reaction of ethylenically unsaturated polymerizable monomers having the moieties attached thereto.

Examples of such monomers are described in E.P. Publication 227,173. Particularly useful monomers are represented by the structure:

$CH_2$ = CR - CONH - COUP

wherein R is hydrogen, halogen or lower alkyl and COUP is a color coupler moiety, many of which are known in the art. Two representative monomers include:

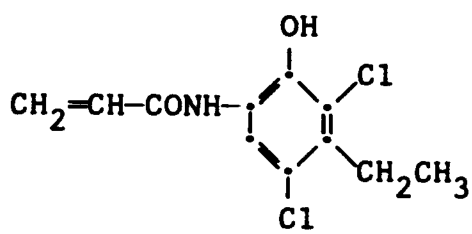

, and

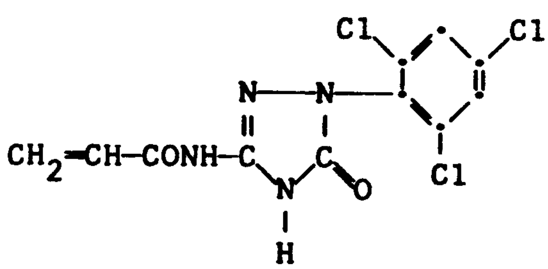

Polymers prepared from such monomers include poly[styrene-*co*-butyl acrylate-*co*-N-3,5-dichloro-4-ethyl-2-hydroxyphenyl)acrylamide)-*co*-methacrylic acid](50:20:20:10 weight ratio), poly[butyl acrylate-*co*-N-3,5-dichloro-4-ethyl-2-hydroxyphenyl)acrylamide-*co*-chloromethylstyrene-*co*-methacrylic acid](20:20:40:20 weight ratio) and poly{butyl acrylate-*co*-N-[1-(2,4,6-trichlorophenyl)-1,2,4-triazoline-5-on-2-yl]acrylamide-*co*-chloromethylstyrene-*co*-methacrylic acid}(20:20:40:20 weight ratio).

The method of the present invention is carried out in two steps, the first of which involves contacting an aqueous suspension of the polymeric particles described above with a carbamoylonium compound described above to produce reactive intermediate polymer particles having intermediate reactive groups in place of the carboxyl groups. This step is carried out at a suitable pH using suitable acids or buffers to provide the desired pH. Generally, the pH is less than 6, but this is not critical as long as the reaction can

proceed. More likely, the pH is between 3.5 and 6. The molar ratio of carbamoylonium compound to the carboxyl groups on the surface of the particles is from 1:100 to 10:1, and preferably from 1:10 to 2:1.

In the second step of the method, the reactive intermediate formed in the first step is contacted with a reactive amine- or sulfhydryl-containing compound having a reactive amine or sulfhydryl group, respectively, which will react with the intermediate reactive group of the reactive intermediate. A covalent linkage is thereby formed between the particles and the reactive compound. The weight ratio of the reactive compound to the polymeric particles is generally from 1:1000 to 1:1, and preferably from 1:100 to 1:10.

This second step can be carried out at a suitable pH such that the desired reaction occurs without premature agglutination. The pH may be varied depending upon the reactants involved and their concentration in the reaction medium. For many proteins and polypeptides, this pH will be greater than 6.

The method of the invention is generally carried out at a temperature of from 10 to 60 °C, and preferably from 15 to 30 °C. The temperature can be the same or different for the two steps of the method.

Further details regarding the method of this invention would be readily apparent to one of ordinary skill in the art from the representative examples which follow.

Example 1: Attachment of Protein to Poly(styrene-co-vinylbenzyl chloride-co-acrylic acid) Particles

A solution of 5.29 g (0.00932 mole) of the carbamoylonium compound 1-(4-morpholinocarbonyl)-4-(2-sulfoethyl)pyridinium hydroxide, inner salt, in 45.71 g of distilled water was added to 50 mℓ of a 4% suspension (pH 3.6) of poly(styrene-co-vinylbenzyl chloride-co-acrylic acid) (molar ratio 85:10:5) particles (average size of about 0.66 micrometer). The resultant mixture had a pH of about 5.0.

A portion of the activated latex containing 100 mg of polymer (dry weight) was incubated at room temperature (about 22 °C) for one hour and a second sample of the same size was incubated three hours. Each was treated with 5 mg of labeled (tritiated) bovine gamma globulin ($^3$H BGG) and brought to a final volume of 30 mℓ with 0.1 molar potassium phosphate (pH 7.0) in 50 mℓ centrifuge tubes. The reactions were continued for five hours at room temperature with end-over-end rotation at 30-35 rpm while attached to a rotating plate mounted at a 45° angle.

A control mixture (Control 1) was similarly prepared by incubating the same amount of a batch of poly(styrene-co-vinylbenzyl chloride-co-acrylic acid) (85:10:5 molar ratio) particles exactly as described above, except it had not been activated with the carbamoylonium compound, to test for nonspecific binding (adsorption). It is known from previous experiments that at low temperatures, there is little, if any, covalent bonding of the bovine gamma globulin protein to the polymer particles via the alternate reaction of the amine groups on the protein with the active chloromethyl groups on the polymer. This latter reaction requires heat and extended reaction times to proceed efficiently.

A second control mixture (Control 2) was prepared by treating 100 mg (dry weight) of latex which had not been activated with the carbamoylonium compound with 5 mg tritiated bovine gamma globulin in 30 mℓ 0.1 molar sodium borate buffer (pH 8.5), with end-over-end rotation as described above for 24 hours at 37 °C.

At the end of the described incubation times, the reaction was quenched by addition of excess bovine serum albumin (100 mg, 20 mg/mℓ in the appropriate buffer). The samples were incubated another 4-18 hours after addition of the albumin.

The total amount of protein bound to the particles was determined by measuring: a) the total cpm (counts per minute) in a 1 mℓ aliquot of the reaction mixture, b) the cpm remaining in the supernatant following centrifugation of a 1 mℓ sample of the reaction mixture and c) the cpm of the latex reagent following repeated washes of the pellet obtained in b). The fraction of the protein which is covalently bound to the particles was determined following incubation of the reagents in the presence of 1% sodium dodecylsulfate surfactant at 37 °C for about 24 hours with end-over-end rotation. The same procedure described above for determining the total amount of bound protein was used to determine the amount of protein covalently bound. The results are reported in the following Tables I and II.

TABLE I

| Sample | Carbamoylonium Reaction Time | % Bound | mg protein/g Polymer |
|---|---|---|---|
| 1 | 1 Hour | 37 | 18 |
| 2 (Control 1) | None | 4 | 2 |
| 3 | 3 Hours | 36 | 18 |
| 4 (Control 2) | None | 22 | 11 |

TABLE II

| After Surfactant Treatment | | |
|---|---|---|
| Sample | % Bound | mg protein/g Polymer |
| 1 | 28 | 14 |
| 2 (Control 1) | 3 | 2 |
| 3 | 27 | 13 |
| 4 (Control 2) | 16 | 8 |

This example demonstrates that bovine gamma globulin can be covalently bound to carboxylated beads using ambient reaction conditions with fast reaction times compared to the alternative reaction schemes of Controls 1 and 2 with better binding efficiency.

Example 2: Preparation and Use of Reagent

Two monoclonal anti-theophylline antibodies were reacted with both carboxylated polymeric particles which had been activated with the carbamoylonium compound used in Example 1 and with particles containing chloromethylstyrene functional groups. The antibodies employed were (1) monoclonal antibodies to theophylline sold by Kallested Laboratories, Inc. (Catalog No. 046, Lot No. W0729, Fill No W0854, clone number 9-49-7A, A8), and (2) monoclonal antibodies specific for theophylline prepared by fusion of SP2/0-Ag14 myeloma cells with splenic lymphocytes from female Balb/C mice immunized with theophylline-bovine serum albumin and which are $\gamma_2$b, kappa isotype having a Ka of approximately 5 x $10^7$ $M^{-1}$ in phosphate buffered saline at room temperature. The amount of antibody bound to the particles was determined in a parallel experiment in which $^3$H bovine gamma globulin was substituted for the anti-theophylline antibody. The amount of active antibody bound to the particles was compared in the enzyme label binding experiment described below.

Carboxylated latex poly(styrene-co-acrylic acid) (99:1 molar ratio) particles were activated with the carbamoylonium compound by suspending a latex containing 500 mg of polymer (dry weight) and 250 mg carbamoylonium compound dissolved in 10 mℓ deionized distilled water in 50 mℓ distilled water at a pH of 5. The latex was stirred for 30 minutes at room temperature and centrifuged. The supernatant was discarded and the latex was resuspended in about 10 mℓ distilled water. Separate samples of the resulting reagent (100 mg of polymer, dry weight) were mixed each with a 3.16 mg sample of one of the two types of antibodies described above in 30 mℓ of buffer (0.05 molar potassium phosphate, pH 7.0). The reactions were incubated with end-over-end rotation at room temperature for about 24 hours. The reactions were stopped by the addition of bovine serum albumin (100 mg, 20 mg/mL) and the incubation was continued for about four hours. The reactions were centrifuged, the supernatants were discarded, and the latices were resuspended in 30 mℓ phosphate buffered saline (pH 7.4) containing 1% TRITON X-100® nonionic surfactant (an octylphenoxy polyethoxy ethanol sold by Rohm and Haas, Co.). The incubations were continued for about 18 hours at 37°C. The latices were then centrifuged, the supernatants discarded, and the resulting pellets were washed twice with phosphate buffered saline and were resuspended in it.

Two samples of a chloromethylstyrene-derived latex [a core-shell copolymer having a core of poly-(styrene-co-divinylbenzene) (99.2:0.8 molar ratio) and a shell of poly(vinylbenzyl chloride-co-divinylbenzene (98.8:1.2 molar ratio)] (100 mg dry polymer in each sample) were incubated with a 3.16 mg sample of one of the above-described antibodies in 30 mℓ of 0.1 molar sodium borate buffer at pH of 8.5. The reactions

were conducted as described above for the carboxylated latex except that the initial incubation was conducted at 37 °C rather than at room temperature.

The amount of antibody bound to each latex preparation was determined by assaying the number of counts for samples run in parallel having $^3H$ bovine gamma globulin bound to core-shell particles having a core of poly(styrene-co-divinylbenzene) and a shell of poly(vinylbenzylchloride-co-divinylbenzene) and particles of poly(styrene-co-acrylic acid) by exactly the same procedures, and in the same amounts, as given above. The relative amount of active antibody in each preparation was determined in an assay in which serial dilutions of the latex ($3.16 \times 10^{-10}$ molar to $1 \times 10^{-6}$ molar theoretical theophylline binding sites based on the mass of antibody bound) were mixed with a fixed concentration of a theophylline-glucose oxidase label ($5 \times 10^{-10}$ molar). The latex dilutions and label were incubated for about 1 hour with constant agitation at room temperature in phosphate buffered saline containing 1% bovine serum albumin. The amount of theophylline-glucose oxidase label remaining in solution following centrifugation was determined and the concentration of theophylline binding sites required to bind 50% of the enzyme label was determined. The results are summarized below:

| Mass Binding Experiment | | |
|---|---|---|
| | % Bound | mg $^3H$ bovine gamma globulin/g Latex |
| Core-Shell Copolymer Latex | 58.3% | 18.4 |
| Poly(styrene-co-acrylic acid) (99:1 molar ratio) | 54.4% | 17.2 |

| Latex-Enzyme Label Titration | | |
|---|---|---|
| Latex | Antibody | nmolar Theoretical theophylline binding sites where 50% of the label is bound |
| Core-Shell Copolymer | 1 | 2.1 |
| | 2 | 11.3 |
| Poly(styrene-co-acrylic acid) (99:1 molar ratio) | 1 | 3.6 |
| | 2 | 2.5 |

This example demonstrates that antibody can be covalently bound to carboxylated beads using ambient reaction conditions according to the present invention at approximately the same efficiency as it can be attached to beads via active halogen groups at elevated temperatures. In addition it demonstrates that the antibody can be attached to activated carboxylated beads with the same degree of preservation of antibody activity as is seen with the alternative protocol of direct linking of the antibodies to the polymer bead via active halogen groups. In several previous experiments it has been shown that more than 50% of the mass of antibody attached to active halogen beads by the procedure described herein remain active towards tritiated theophylline. Therefore it is expected that the same would be observed for the antibody attached to activated carboxyl beads.

Example 3: Comparison with Carbodiimide Attachment

This example compares the method of the present invention to a method of attaching a protein to a particle using known carbodiimide chemistry, and compares the use of the resulting reagents in an assay for phenobarbital.

A monoclonal antibody to phenobarbital was prepared at Eastman Kodak Co. by immunization of Balb/c mice with a conjugate of phenobarbital-human serum albumin. Spleens of the immunized mice were fused with myeloma (SP2/0-Ag 14) cells to generate the hybridomas. This preparatory method used known procedures.

The antibody so prepared was covalently attached to polymeric particles having pendant carboxyl groups on the outer surfaces. The particles were composed of poly(styrene-co-methacrylic acid) (90:10 molar ratio). The mass of antibody bound to the particles was determined in a parallel experiment in which tritiated bovine gamma globulin was used in place of the anti-phenobarbital antibody. The amount of active protein bound to the particles was compared in the enzyme label binding experiment as described in

Example 2 above.

One sample of the polymeric particles described above (30 mg dry weight) was mixed with 1-(4-morpholinocarbonyl)-4-(2-sulfoethyl)pyridinium hydroxide, inner salt (16 mg, 1.5 mmole/g beads) in 10 ml of 0.1 molar 2-(N-morpholino)ethanesulfonic acid buffer (pH 6) for 10 minutes, followed by the addition of the anti-phenobarbital antibody (0.3 mg). A second sample of the polymeric latex was mixed with the same inner salt, followed by addition of 1.5 mg of the anti-phenobarbital antibody.

A third sample of the same latex was mixed with 1-cyclohexyl-3-(2-morpholinoethyl)carbodiimide metho-p-toluenesulfonate (19.1 mg, 1.5 mmole/g beads) for ten minutes, followed by the addition of 0.3 mg of the anti-phenobarbital antibody. A fourth sample of the latex particles was mixed with the carbodiimide, followed by the addition of 1.5 mg of antibody. These samples are identified as Controls 1 and 2 in Tables III and IV below.

The attachment reactions were carried out by incubation of the mixtures for 24 hours with end-over-end rotation at room temperature. The reactions were stopped by the addition of bovine serum albumin (30 mg, 30 mg/ml), and incubation was then continued for an additional four hours. The reaction mixtures were centrifuged, the supernatant discarded, and the pellets washed once with phosphate buffered saline solution (pH 7.4), and then resuspended in the saline solution.

The mass of antibody bound to each latex preparation was determined by assaying the number of radioactive counts for samples run in parallel having tritiated bovine gamma globulin bound to the particles as described in Example 1. The covalent/total ratio was calculated following incubation with sodium dodecylsulfate surfactant as described in Example 1. The results of the mass binding experiment are summarized in Tables III and IV below.

The relative amount of active antibody in each preparation was determined in an enzyme label binding experiment similar to the one described in Example 2. The present example demonstrates that the anti-phenobarbital antibody can be covalently bound to the polymeric particles using the carbamoylonium compound at very high efficiency. It also demonstrates that the antibody can be attached to form a useful immunological reagent. At the low antibody/polymer bead ratio, a four-fold lower concentration of immobilized antibody reagent of the present invention was required to bind 50% of the labeled antigen than for the Control reagent (compare 86 nmolar theoretical phenobarbital binding sites of the Control to 19 nmolar for the invention at 0.3 mg protein added). This suggests a four times greater retention of antibody activity for the method of the present invention over the carbodiimide chemistry of the prior art.

TABLE III
(Mass Binding Experiment)

| Attachment Method | Labeled Protein Used (mg) | % Bound | mg $^3H$ Protein/ g polymer | Covalent/ Total |
|---|---|---|---|---|
| Example 3 | 0.3 | 82.8 | 8.28 | 0.99 |
| Example 3 | 1.5 | 82.8 | 41.4 | 0.93 |
| Control 1 | 0.3 | 90.7 | 9.07 | 0.94 |
| Control 2 | 1.5 | 73.8 | 36.9 | 0.80 |

TABLE IV

(Latex-Enzyme Label Titration)

| Attachment Method | Labeled Protein Used (mg) | Theoretical Phenobarbital Binding Sites to Bind 50% of Label (nmolar) |
|---|---|---|
| Example 3 | 0.3 | 19 |
| Example 3 | 1.5 | 10 |
| Control 1 | 0.3 | 86 |
| Control 2 | 1.5 | 10 |

Example 4: Attachment of Diamine to Polymeric Particles

This example illustrates the practice of the present invention by the attachment of diamines to carboxylated polymeric particles using a carbamoylonium compound. The importance of this feature of the invention resides in the fact that it is difficult to prepare particles having reactive amine moieties on the outer surface using known polymerization methods of amine-containing monomers.

A portion (3 g dry weight) of poly(styrene-co-acrylic acid)(90:10 molar ratio) suspended in deionized distilled water (100 ml) was combined with 1.5 g of 1-(4-morpholinocarbonyl)-4-(2-sulfoethyl)pyridinium hydroxide, inner salt dissolved in deionized distilled water (60 ml) and the volume was brought to 300 ml with distilled water. The reaction mixture was stirred at room temperature for 30 minutes, and then centrifuged at 10 °C and 6000 rpm for 30 minutes. The supernatant was discarded, and the beads were resuspended in 90 ml of deionized distilled water. The latex containing thusly activated polymeric particles was divided into three portions and each portion was reacted with an excess of one of the three diamines

described below.

Reaction A: Diamine A, Hexanediamine (567 mg), was dissolved in 30 ml of 0.1 molar sodium borate buffer (pH 8) and the pH was adjusted to 8. This solution was added to 30 ml of the activated latex and the volume was brought to 100 ml with borate buffer.

Reaction B: Diamine B, L-lysine•1HCl (548 mg), was dissolved and reacted as described for Reaction A.

Reaction C: Diamine C, L-lysyl-L-lysine•2HCl (260 mg), was also dissolved and reacted as described for Reaction A.

The three reactions described above were continued for four hours at room temperature and end-over-end rotation. Each reaction mixture was then placed in a dialysis bag, and dialyzed against deionized distilled water for about 24 hours. They were then centrifuged for 30 minutes at 6000 rpm and the supernatant was discarded. Each reaction product was then resuspended in 100 ml of deionized distilled water, and the centrifugation and resuspension process repeated twice more. The resulting reaction products contained the respective diamine covalently attached to the latex particles.

Each reaction product prepared above was reacted with an IgG protein having oxidized aldehyde groups in the following manner:

The IgG was first dialyzed into sodium phosphate buffer (0.01 molar, pH 6) containing sodium chloride (0.15 molar). The IgG protein used was tritiated bovine gamma globulin ($^3$H BGG). The carbohydrate of the protein was oxidized with $NaIO_4$ at a final concentration of 30 mmolar for 1 hour at room temperature. Glycerol was added at a final concentration of 100 mmolar to stop the reaction and excess reagents were removed by passage of the reaction through a commercially available Pharmacia PD-10 desalting column using the manufacturer's instructions.

A portion (30 mg dry weight) of each of the three reaction products described above was combined with 1.5 mg of the oxidized protein and brought to a final volume of 9 ml with 0.01 molar sodium phosphate buffer (pH 6) containing 0.15 molar sodium chloride in a 15 ml centrifuge tube. The resulting reactions were continued for five hours at room temperature with end-over-end rotation. A 1 ml solution of $NaBH_3CN$ (100 mmolar) was added to each reaction and the rotation was continued for an additional 16 hours at room temperature.

A Control material (Control 1) was similarly prepared by substituting a portion of the original carboxylated latex for the latex containing diamine moieties. Three additional Controls (2-4) were similarly prepared by substituting unoxidized tritiated bovine gamma globulin for the oxidized protein in the reactions with each diamine latex. Unoxidized tritiated bovine gamma globulin was also added to the original carboxylated latex in Control 5.

At the end of the described reactions, each reaction was quenched by the addition of excess bovine serum albumin (30 mg, 30 mg/ml in buffer). The samples were then incubated another four hours after addition of the quenching protein.

The total amount of antibody bound to the latex particles was determined by the procedures described in Example 2 above. The fraction of the $^3$H BGG which is covalently bound to the particles was determined following incubation in the presence of 1% sodium dodecyl sulfate as described in Example 2 above. The results are provided in Table V below.

TABLE V

| Polymeric Reaction Product | IgG | Total Bound (mg BGG/g Polymer) | Covalent Bound | Covalent/ Total Ratio |
|---|---|---|---|---|
| Invention: | | | | |
| Diamine A | Oxidized | 28 | 20 | 0.71 |
| Diamine B | Oxidized | 29 | 21 | 0.72 |
| Diamine C | Oxidized | 27 | 23 | 0.85 |
| Controls: | | | | |
| Control 1 (No Diamine) | Oxidized | 25 | 11 | 0.44 |
| Control 2 (Diamine A) | Unoxidized | 22 | 1.2 | 0.05 |
| Control 3 (Diamine B) | Unoxidized | 22 | 2.5 | 0.11 |
| Control 4 (Diamine C) | Unoxidized | 21 | 3.9 | 0.19 |
| Control 5 (No Diamine) | Unoxidized | 25 | 1.9 | 0.08 |

These results with Diamines A-C demonstrate that the diamines reacted with the carboxyl groups on the polymeric particles following activation by the carbamoylonium compound according to the present invention. The resulting amine moieties on the particles then reacted with the oxidized aldehyde groups on the tritiated IgG. As shown by the data, aldehyde groups must be present on the protein for substantial binding to the amine moieties on the particles. Where the groups were not oxidized (Controls 2-5), only a small fraction of the protein was covalently attached to the particles. In addition, sufficient amine moieties must be present on the particles for significant covalent attachment. Little covalent attachment is seen with the carboxylated particles alone (Control 1).

Example 5: Attachment of Protein to Particles Prepared from Monomeric Color Couplers

Polymeric particles of poly[styrene-co-butyl acrylate-co-N-(3,5-dichloro-4-ethyl-2-hydroxyphenyl)-acrylamide-co-methacrylic acid)(50:20:20:10 weight ratio) were prepared in the following manner:

Nitrogen-purged distilled water (1000 g) was added to a flask fitted with a stirrer, condenser and nitrogen supply, and placed in a water bath heated to 60°C. Styrene (50 g), n-butyl acrylate (20 g) and N-(3,5-dichloro-4-ethyl-2-hydroxyphenyl)acrylamide (20 g) and acetone (200 ml) were mixed and added to the flask. An initiator combination of $K_2S_2O_8$ (2 g) and $K_2S_2O_5$ (1 g) was added to the flask and the reaction was allowed to proceed for 15 minutes. Methacrylic acid (20 g) was then added and the reaction continued for 18 hours to provide a latex of polymeric particles.

To develop the color coupler moieties of the polymeric particles a color developer composition of the following components was then added, along with 200 ml of a 10% $K_2S_2O_8$ solution:
triethanolamine (11 ml), benzyl alcohol (14.2 ml), lithium chloride (2.1 g), potassium bromide (0.6 g), hydroxylamine sulfate (3.2 g), potassium sulfite (45% solution, 2.8 ml), 1-hydroxyethylene-1,1-di-phosphoric acid (60%, 0.8 ml), 4-amino-3-methyl-N-ethyl-N-($\beta$-methanesulfonamidoethyl)aniline sulfate hydrate (4.35 g), potassium carbonate (anhydrous, 28 g), stilbene whitening agent (0.6 g) surfactant (1 ml) and water to make 1 liter (pH 10.8). The resulting colored beads were dialyzed for 24 hours in a continuous dialysis bath against distilled water, and residual monomers was removed in a rotary evaporator at 80°C.

The carbamoylonium compound 1-(4-morpholinocarbonyl)-4-(2-sulfoethyl)pyridinium hydroxide, inner salt (0.15 mmolar) was added to 100 mg of the developed particles described above in 30 ml of 0.1 molar 2-(4-morpholine)ethanesulfonic acid (pH 6) in a 50 ml polypropylene centrifuge tube. This suspension was rotated for 10 minutes at 30-35 rpm while attached to a rotating plate mounted at a 45° angle. Tritiated bovine gamma globulin (1 mg) was added to the reaction mixture and rotation was continued for an additional 24 hours. Reaction was quenched by addition of excess bovine serum albumin (100 mg, 50 mg/ml in the buffer), and the sample was incubated for an additional 24 hours.

The total amount of antibody bound to the particles was determined by measuring the counts per minute from the radioisotope remaining in the supernatant following centrifugation of a 1 ml sample of the reaction and subtracting this amount from the total theoretical counts per minutes input into each reaction. It was determined that 93% of the protein was bound to the particles (9.3 mg protein bound per g of polymer).

## Claims

**1.** A method for attaching a reactive amine- or sulfhydryl-containing compound to polymeric particles through pendant carboxy groups,

the method characterized wherein it comprises:

A. contacting (1) an aqueous suspension of polymeric particles having carboxyl groups on the surface thereof and prepared from one or more ethylenically unsaturated polymerizable monomers, at least one of which contains pendant carboxyl groups with (2) a carbamoylonium compound to produce reactive intermediate polymer particles having pendant intermediate reactive groups, and

B. contacting the reactive intermediate polymer particles produced in step A with a reactive amine- or sulfhydryl-containing compound having a reactive amine or sulfhydryl group, respectively, which reacts with the intermediate reactive groups to form a covalent linkage between the particles and the reactive compound, and

the carbamoylonium compound having the structure

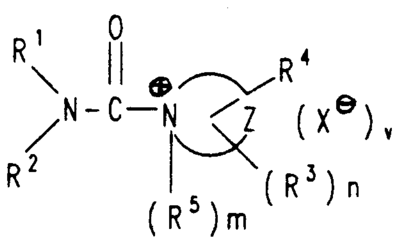

wherein

Z represents the atoms necessary to complete a substituted or unsubstituted 5- or 6-membered heterocyclic aromatic ring,

m and n are independently 0 or 1,

$R^1$ and $R^2$ are independently of each other, substituted or unsubstituted alkyl, or substituted or

unsubstituted aryl, or $R^1$ and $R^2$ together represent the atoms necessary to complete a substituted or unsubstituted piperidine, piperazine or morpholine ring,

$R^3$ is a hydrogen atom, a substituted or unsubstituted alkyl, or the group

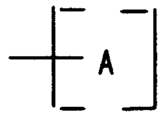

wherein A represents the polymerized vinyl backbone of a homo- or copolymer formed from one or more ethylenically unsaturated polymerizable compounds such that the molecular weight of said homo- or copolymer is greater than 1000,

$R^4$ is a hydrogen atom, a substituted or unsubstituted alkyl, or when Z represents the atoms necessary to complete a pyridinium ring and n is 0, $R^4$ is selected from the following groups:

(a) $-NR^6-CO-R^7$ wherein $R^6$ is hydrogen or substituted or unsubstituted alkyl, $R^7$ is hydrogen, substituted or unsubstituted alkyl or $-NR^8R^9$ wherein $R^8$ and $R^9$ are independently hydrogen or substituted or unsubstituted alkyl,

(b) $-(CH_2)_q-NR^{10}R^{11}$ wherein $R^{10}$ is $-CO-R^{12}$, $R^{11}$ is hydrogen or substituted or unsubstituted alkyl, $R^{12}$ is hydrogen, substituted or unsubstituted alkyl or $-NR^{13}R^{14}$ wherein $R^{13}$ is substituted or unsubstituted alkyl or substituted or unsubstituted aryl, $R^{14}$ is hydrogen, substituted or unsubstituted alkyl or substituted or unsubstituted aryl, and q is 1 to 3,

(c) $-(CH_2)_r-CONR^{15}R^{16}$ wherein $R^{15}$ is hydrogen, substituted or unsubstituted alkyl or substituted or unsubstituted aryl, $R^{16}$ is hydrogen or substituted or unsubstituted alkyl, or $R^{15}$ and $R^{16}$ together represent the atoms necessary to complete a 5- or 6-membered aliphatic ring, and r is 0 to 3,

(d) 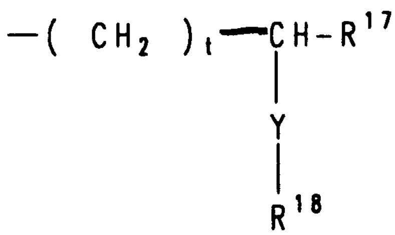

wherein $R^{17}$ is hydrogen or substituted or unsubstituted alkyl, Y is oxy or $-NR^{19}-$, $R^{18}$ is hydrogen, substituted or unsubstituted alkyl, $-CO-R^{20}$ or $-CO-R^{21}$ wherein $R^{19}$, $R^{20}$ and $R^{21}$ are independently hydrogen or substituted or unsubstituted alkyl, and t is 2 or 3, and

(e) $-R^{21}X'^{\theta}$ wherein $R^{21}$ is substituted or unsubstituted alkylene, and $X'^{\theta}$ is a covalently bonded anionic group so as to form an inner salt group with the pyridinium ring,

$R^5$ is substituted or unsubstituted alkyl, or substituted or unsubstituted aryl provided that m is 0 when the nitrogen atom to which $R^5$ is bound is attached to the remainder of the ring through a double bond, and

$X^{\theta}$ is an anion, and v is 0 or 1, provided that it is 0 only when $R^4$ is $-R^{21}X'^{\theta}$.

2. The method as claimed in claim 1 wherein either the polymeric particles used in step A or the reactive amine- or sulfhydryl-containing compound used in step B has a detectable tracer compound associated therewith.

3. The method as claimed in either of claims 1 or 2 wherein the reactive amine- or sulfhydryl-containing compound is a polypeptide or protein.

4. The method as claimed in any of claims 1 to 3 wherein the carbamoylonium compound is present in a molar ratio to the total measured carboxylic acid level in the polymeric particles from 1:100 to 10:1.

5. The method as claimed in any of claims 1 to 4 carried out at a temperature of from 10°C to 60°C.

6. The method as claimed in any of claims 1 to 5 wherein the carbamoylonium compound has the defined

structure wherein $R^1$ and $R^2$ together represent the atoms necessary to complete a piperidine, piperazine or morpholine ring.

7. The method as claimed in any of claims 1 to 6 wherein the carbamoylonium compound has the defined structure wherein $R^1$ and $R^2$ together represent the atoms necessary to complete a morpholine ring, Z represents the atoms necessary to complete a pyridinium ring, $R^4$ is $-R^{21}X'^{\ominus}$, and m, n and v are each 0.

8. The method as claimed in any of claims 1 to 7 wherein the carbamoylonium compound is 1-(4-morpholinocarbonyl)-4-(2-sulfoethyl)pyridinium hydroxide, inner salt, or 1-(4-morpholinocarbonyl)-pyridinium chloride.

9. The method as claimed in any of claims 1 to 8 wherein the reactive amine- or sulfhydryl-containing compound is an immunologically reactive species.

10. The method as claimed in claim 9 wherein the immunologically reactive species is an antibody to any of a Streptococcus A antigen, a chlamydial antigen, a gonococcal antigen, human chorionic gonadotropin, human leutinizing hormone, a herpes virus, a drug or hormone, or it is a HTLV or HIV antigen.

## Revendications

1. Méthode pour relier un composé contenant des groupes réactifs amine ou sulfhydryle à des particules polymères par l'intermédiaire d'un groupe carboxy, caractérisée en ce qu'elle comprend les étapes de :
   A. mise en contact (1) d'une suspension aqueuse de particules polymères ayant des groupes carboxy en surface et préparées à partir d'un ou plusieurs monomères polymérisables à insaturations éthyléniques, au moins un de ces polymères comprenant des groupes carboxyle non-inclus dans le squelette du polymère, avec un composé carbamylonium (2) pour produire des particules polymères réactives intermédiaires ayant des groupes réactifs intermédiaires non inclus dans le squelette du polymère, et
   B. mise en contact des particules polymères intermédiaires réactives obtenues pendant l'étape A avec un composé réactif contenant respectivement un groupe réactif amine ou sulfhydryle qui réagit avec les groupes réactifs intermédiaires pour former une liaison covalente entre les particules et le composé réactif, et

   le composé carbamylonium ayant la structure :

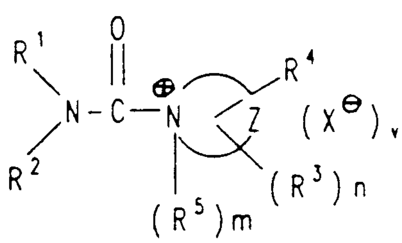

où Z représente les atomes nécessaires pour former un hétérocycle aromatique à 5 ou 6 chaînons, m et n sont indépendamment 0 ou 1,

$R^1$ et $R^2$ sont, chacun séparément, un groupe alkyle ou aryle substitué ou non, ou $R^1$ et $R^2$, pris ensemble, représentent les atomes nécessaires pour former un cycle pipéridine, pipérazine ou morpholine,

$R^3$ est un atome d'hydrogène, un groupe alkyle substitué ou non, ou le groupe

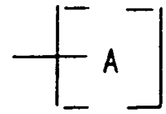

où A représente le squelette vinylique polymérisé d'un homopolymère ou d'un copolymère obtenu à partir d'un ou plusieurs composés polymérisables à insaturations éthyléniques, tels que le poids moléculaire de l'homopolymère ou du copolymère est supérieur à 1000,

$R^4$ est un atome d'hydrogène, un groupe alkyle substitué ou non, ou lorsque Z représente les atomes nécessaires pour former un cycle pyridinium et n est 0, $R^4$ est choisi parmi les groupes suivants :

(a) $-NR^6-CO-R^7$ où $R^6$ est un atome d'hydrogène ou un groupe alkyle substitué ou non, $R^7$ est un atome d'hydrogène, un groupe alkyle substitué ou non ou $-NR^8R^9$ ou $R^8$ et $R^9$ sont chacun séparément, un atome d'hydrogène ou un groupe alkyle substitué ou non,

(b) $-(CH_2)_q-NR^{10}R^{11}$ où $R^{10}$ est $-CO-R^{12}$, $R^{11}$ est un atome d'hydrogène ou un groupe alkyle substitué ou non, $R^{12}$ est un atome d'hydrogène, un groupe alkyle substitué ou non, ou $-NR^{13}R^{14}$ où $R^{13}$ est un groupe aryle ou alkyle substitué ou non, $R^{14}$ est un atome d'hydrogène, un groupe aryle ou alkyle substitué ou non, et q est compris entre 1 et 3,

(c) $-(CH_2)_r-CONR^{15}R^{16}$ où $R^{15}$ est un atome d'hydrogène, un groupe aryle ou alkyle substitué ou non, $R^{16}$ est un atome d'hydrogène ou un groupe alkyle substitué ou non, ou $R^{15}$ et $R^{16}$, pris ensemble, représentent les atomes nécessaires pour former un cycle aliphatique à 5 ou 6 chaînons, et r est compris entre 0 et 3

(d) 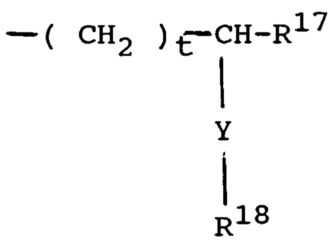

où $R^{17}$ est un atome d'hydrogène ou un groupe alkyle substitué ou non, Y est un groupe oxy ou $-NR^{19}-$,

$R^{18}$ est un atome d'hydrogène, un groupe alkyle substitué ou non, $-CO-R^{20}$ ou $-CO-NHR^{21}$ où $R^{19}$, $R^{20}$ et $R^{21}$ sont chacun séparément un atome d'hydrogène ou un groupe alkyle substitué ou non et t est 2 ou 3, et

(e) $-R^{21}X'^{\theta}$ où $R^{21}$ est un groupe alkylène substitué ou non, et $X'^{\theta}$ est un groupe anionique lié de façon covalente pour former un sel interne avec le cycle pyridinium,

$R^5$ est un groupe alkyle ou aryle substitué ou non avec la condition supplémentaire que m est 0 si l'atome d'azote sur lequel $R^5$ est lié, est attaché sur le reste du cycle au moyen d'une double liaison, et

$X^{\theta}$ est un anion, et v est 0 ou 1 avec la condition supplémentaire que si v est 0 alors $R^4$ est $-R^{21}X'^{\theta}$.

**2.** Méthode selon la revendication 1 dans laquelle les particules polymères utilisées dans l'étape A ou le composé contenant des groupes réactifs amine ou sulfhydryle utilisé dans l'étape B sont associés avec un composé traceur détectable.

**3.** Méthode selon l'une quelconque des revendications 1 ou 2 dans laquelle le composé contenant un groupe réactif amine ou sulfhydryle est un polypeptide ou une protéine.

**4.** Méthode selon l'une quelconque des revendications 1 à 3 dans laquelle le rapport molaire de composé carbamylonium sur la quantité totale mesurée d'acide carboxylique dans les particules polymères est compris entre 1:100 et 10:1.

**5.** Méthode selon l'une quelconque des revendications 1 à 4 mise en oeuvre à une température comprise entre 10 ° C et 60 ° C.

**6.** Méthode selon l'une quelconque des revendications 1 à 5 dans laquelle le composé carbamylonium a la structure définie lorsque $R^1$ et $R^2$, pris ensemble, représentent les atomes nécessaires pour former un cycle pipéridine, pipérazine ou morpholine.

**7.** Méthode selon l'une quelconque des revendications 1 à 6 dans laquelle le composé carbamylonium a

la structure définie lorsque $R^1$ et $R^2$, pris ensemble, représentent les atomes nécessaires pour former un cycle morpholine, Z représente les atomes nécessaires pour former un cycle pyridinium, $R^4$ est $-R^{21}X'^{\ominus}$, et m, n et v sont chacun 0.

8. Méthode selon l'une quelconque des revendications 1 à 7 dans laquelle le composé carbamylonium est l'hydroxyde de 1-(4-morpholinocarbonyl)-4-(2-sulfoéthyl)pyridinium, un sel interne ou un chlorure de 1-(4-morpholinocarbonyl) pyridinium.

9. Méthode selon l'une quelconque des revendications 1 à 8 dans laquelle le composé contenant un groupe réactif amine ou sulfhydryle est une espèce immunologiquement réactive.

10. Méthode selon la revendication 9 dans laquelle l'espèce immunologiquement réactive est un anticorps d'un des composés suivants : l'antigène du streptocoque A, un antigène chlamydial, un antigène gonocoque, une gonatropine chorionique humaine, une hormone leutinisante humaine, un virus de l'herpès, un produit pharmaceutique ou une hormone, ou un antigène HTLV ou HIV.

**Patentansprüche**

1. Verfahren zum Verknüpfen einer eine reaktionsfähige Aminoder Sulfhydrylgruppe enthaltenden Verbindung mit polymeren Teilchen über abstehende Carboxylgruppen, dadurch gekennzeichnet, daß das Verfahren umfaßt:

   A. Kontaktieren von (1) einer wäßrigen Suspension von polymeren Teilchen mit Carboxylgruppen an der Oberfläche und hergestellt aus einem oder mehreren ethylenisch ungesättigten polymerisierbaren Monomeren, von denen mindestens eines abstehende Carboxylgruppen aufweist, mit (2) einer Carbamoyloniumverbindung unter Erzeugung reaktionsfähiger Zwischen-Polymerteilchen mit abstehenden reaktionsfähigen Zwischengruppen, und

   B. Kontaktieren der reaktionsfähigen Zwischen-Polymerteilchen, die in der Stufe A erhalten wurden, mit einer eine reaktionsfähige Amin- oder Sulfhydrylgruppe enthaltenden Verbindung, die mit den reaktionsfähigen Zwischengruppen unter Bildung einer covalenten Bindung zwischen den Teilchen und der reaktionsfähigen Verbindung reagiert, wobei

   die Carbamoyloniumverbindung der folgenden Strukturformel entspricht:

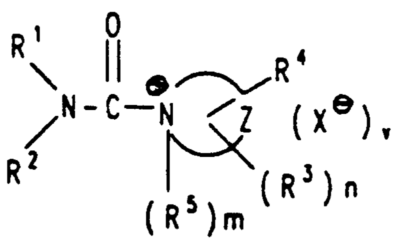

in der bedeuten:

| | |
|---|---|
| Z | die zur Vervollständigung eines 5- oder 6-gliedrigen heterocyclischen aromatischen Ringes erforderlichen Atome, |
| m und n | unabhängig voneinander 0 oder 1, |
| $R^1$ und $R^2$ | unabhängig voneinander Alkyl oder Aryl oder $R^1$ und $R^2$ gemeinsam die Atome, die zur Vervollständigung eines Piperidin-, Piperazin- oder Morpholinringes erforderlich sind; |
| $R^3$ | ein Wasserstoffatom, Alkyl oder die Gruppe: |

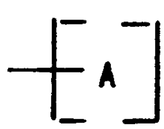

in der A die polymerisierte Vinylkette eines Homo- oder Copolymeren darstellt, das hergestellt ist aus einem oder mehreren ethylenisch ungesättigten polymerisierbaren Verbindungen, derart, daß das Molekulargewicht des Homo- oder Copolymeren größer

als 1000 ist,

$R^4$ ein Wasserstoffatom, Alkyl, oder falls Z für die Atome steht, die zur Vervollständigung eines Pyridiniumringes erforderlich sind und n gleich 0 ist, steht $R^4$ für eine Gruppe, ausgewählt aus den folgenden Gruppen:

(a) $-NR^6-CO-R^7$, worin $R^6$ für Wasserstoff oder Alkyl steht, und worin $R^7$ Wasserstoff, Alkyl oder $-NR^8R^9$ ist, worin $R^8$ und $R^9$ unabhängig voneinander für Wasserstoff oder Alkyl stehen,

(b) $-(CH_2)_q-NR^{10}R^{11}$, worin $R^{10}$ für $-CO-R^{12}$ steht, $R^{11}$ gleich Wasserstoff oder Alkyl ist, $R^{12}$ die Bedeutung von Wasserstoff, Alkyl oder $-NR^{13}R^{14}$ hat, worin $R^{13}$ gleich Alkyl oder Aryl ist, $R^{14}$ für Wasserstoff, Alkyl oder Aryl steht und q gleich 1 bis 3 ist,

(c) $-(CH_2)_r-CONR^{15}R^{16}$, worin $R^{15}$ gleich Wasserstoff, Alkyl oder Aryl ist, $R^{16}$ für Wasserstoff oder Alkyl steht oder $R^{15}$ und $R^{16}$ gemeinsam für die Atome stehen, die zur Vervollständigung eines 5- oder 6-gliedrigen aliphatischen Ringes erforderlich sind, und r gleich 0 bis 3 ist,

(d) 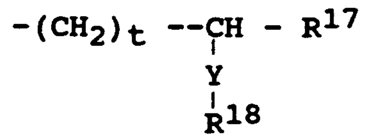

worin $R^{17}$ gleich Wasserstoff oder Alkyl ist, Y für Oxy oder $-NR^{19}-$ steht, $R^{18}$ Wasserstoff, Alkyl, $-CO-R^{20}$ oder $-CONHR^{21}$ darstellt, worin $R^{19}$, $R^{20}$ und $R^{21}$ unabhängig voneinander Wasserstoff oder Alkyl darstellen und t gleich 2 oder 3 ist, und

(e) $-R^{21}X'^{\theta}$, worin $R^{21}$ für Alkylen steht und $X'^{\theta}$ eine covalent gebundene anionische Gruppe darstellt, unter Bildung einer inneren Salzgruppe mit dem Pyridiniumring,

$R^5$ gleich Alkyl oder Aryl, vorausgesetzt, daß m gleich 0 ist, wenn das Stickstoffatom, an das $R^5$ gebunden ist, an den Rest des Ringes durch eine Doppelbindung gebunden ist, und

$X'^{\theta}$ ein Anion, und v gleich 0 oder 1, wobei gilt, daß v nur 0 ist, wenn $R^4$ für die Gruppe der Formel $-R^{21}X'^{\theta}$ steht.

2. Verfahren nach Anspruch 1, in dem entweder die in der Stufe A verwendeten Polymerteilchen oder die in Stufe B verwendete reaktionsfähige Verbindung mit Amin- oder Sulfhydrylgruppen eine ermittelbare Spurenverbindung aufweist, die mit den Teilchen oder der reaktionsfähigen Verbindung verbunden ist.

3. Verfahren nach einem der Ansprüche 1 oder 2, in dem als reaktionsfähige Verbindung mit Amin- oder Sulfhydrylgruppen ein Polypeptid oder Protein verwendet wird.

4. Verfahren nach einem der Ansprüche 1 bis 3, in dem die Carbamoyloniumverbindung in einem Molverhältnis zur gesamten Carboxylsäuremenge in den Polymerteilchen von 1:100 bis 10:1 vorliegt.

5. Verfahren nach einem der Ansprüche 1 bis 4, dadurch gekennzeichnet, daß es bei einer Temperatur von 10 °C bis 60 °C durchgeführt wird.

6. Verfahren nach einem der Ansprüche 1 bis 5, in dem die Carbamoyloniumverbindung die angegebene Struktur hat, in der $R^1$ und $R^2$ gemeinsam die Atome darstellen, die zur Vervollständigung eines Piperidin-, Piperazin- oder Morpholinringes erforderlich sind.

7. Verfahren nach einem der Ansprüche 1 bis 6, in dem die Carbamoyloniumverbindung der angegebenen Formel entspricht, in der $R^1$ und $R^2$ gemeinsam für die Atome stehen, die zur Vervollständigung eines Pyridiniumringes erforderlich sind, $R^4$ gleich $-R^{21}X'^{\theta}$ ist und m, n und v gleich 0 sind.

8. Verfahren nach einem der Ansprüche 1 bis 7, in dem die Carbamoyloniumverbindung ein inneres Salz von 1-(4-Morpholinocarbonyl)-4-(2-sulfoethyl)pyridiniumhydroxid oder 1-(4-Morpholinocarbonyl)-pyridiniumchlorid ist.

**9.** Verfahren nach einem der Ansprüche 1 bis 8, in dem die reaktionsfähige Verbindung mit den Amin- oder Sulfhydrylgruppen einer immunologisch reaktionsfähigen Gattung angehört.

**10.** Verfahren nach Anspruch 9, in dem die immunologisch reaktionsfähige Gattung ein Antikörper für ein beliebiges Streptococcus A Antigen ist, ein Chlamydien-Antigen, ein gonococciales Antigen, ein menschliches chlorionisches Gonadotropin, ein menschliches leutinisierendes Hormon, ein Herpes-Virus, ein Arzneimittel oder Hormon oder ein HTLV- oder HIV-Antigen.